# EUROPEAN PATENT SPECIFICATION

(11) **EP 4 397 280 B1**
(45) Date of publication and mention of the grant of the patent: **04.03.2026**
(21) Application number: 24178251.5
(22) Date of filing: 04.10.2021
(51) Int. Cl.: A61F 2/24, A61M 25/00, A61F 2/95

(54) **TRANSFER SLEEVE FOR USE WITH A MEDICAL DEVICE DELIVERY SYSTEM**
TRANSFERHÜLSE ZUR VERWENDUNG MIT EINEM ZUFÜHRSYSTEM EINER MEDIZINISCHEN VORRICHTUNG
MANCHON DE TRANSFERT DESTINÉ À ÊTRE UTILISÉ AVEC UN SYSTÈME DE DISTRIBUTION DE DISPOSITIF MÉDICAL

(30) Priority: 05.10.2020 US 202063087549 P; 30.09.2021 US 202117490177
(43) Date of publication of application: 10.07.2024
(62) Divisional of application: 21200728.0
(73) Proprietor: Medtronic Vascular, Inc., Santa Rosa, CA 95403 (US)
(72) Inventor: PFEIFFER, Ronja Fiona, Santa Rosa (US); FRISBY, Pariac, Santa Rosa (US)
(74) Representative: Zimmermann & Partner Patentanwälte mbB

(56) References cited:
- US-A1- 2011 208 284
- US-A1- 2015 305 863

## Description

### CROSS-REFERENCE TO RELATED APPLICATIONS

This application claims the benefit under 35 U.S.C. § 119(e) of the filing date of U.S. Provisional Application No. 63/087,549, filed October 5, 2020

### FIELD

The present technology is generally related to medical devices. And, more particularly, to transfer sleeves for delivery systems for stents, prosthetic heart valves and other implantable medical devices.

### BACKGROUND

Patients suffering from various medical conditions or diseases may require surgery to install an implantable medical device. For example, valve regurgitation or stenotic calcification of leaflets of a heart valve may be treated with a prosthetic heart valve. A traditional surgical procedure to implant the prosthetic heart valve requires a sternotomy and a cardiopulmonary bypass, which creates significant patient trauma and discomfort. Traditional surgical procedures may also require extensive recuperation times and may result in life-threatening complications. One alternative to a traditional surgical procedure is delivering implantable medical devices using minimally-invasive techniques. For example, a prosthetic heart valve can be percutaneously and transluminally delivered to an implant location. In such methods, the prosthetic heart valve can be compressed or crimped into a delivery catheter for insertion within a patient's vasculature, advanced to the implant location, and re-expanded to be deployed at the implant location.

During certain procedures, components of a delivery system for the implantable medical device may need to be limited in size and shape due to a patient's anatomy. For example, a capsule of a delivery system used in a transseptal mitral valve replacement procedure may need to be limited in length due to limited space in the heart chambers for tracking and deployment. Due to the limited length of the capsule, a portion of the implantable medical device (*e.g.,* a mitral prosthetic heart valve) remains exposed, *i.e.,* is not contained within the capsule. When using implantable medical devices such as a mitral prosthetic heart valve with a tissue valve, the implantable medical device is loaded into the delivery system just prior to the medical procedure at the site of the medical procedure. During a typical loading procedure, the implantable medical device is loaded onto the delivery system under saline. A method of maintaining a flushed status of the implant of the system and the implant during transfer from the loading bath to insertion into the patient is required without compromising the working length of the catheter.

US 2011/208284 A1 relates to a protective sleeve for a medical device.

US 2015/305863 A1 describes intravascular introducer devices.

### Summary

The claimed invention is defined in independent claim 1. Further aspects and preferred embodiments are defined in the dependent claims.

The techniques and devices of this disclosure generally relate to transfer sleeves for protecting an implantable medical device installed onto a delivery system.

In one aspect, corresponding to the claimed invention, the present disclosure provides a transfer sleeve for use with a delivery system for an implantable medical device. The transfer sleeve includes a proximal portion defining a proximal channel. The proximal channel has a cross-sectional area that allows a shaft of the delivery system to fit within the proximal channel of the proximal portion. The transfer sleeve also includes a distal portion defining a distal channel. The distal channel has a cross-section area that allows the shaft of the delivery system to fit within the distal channel of the distal portion. Further, the transfer sleeve includes a body portion coupled between the proximal portion and the distal portion and defining a body cavity. The body portion is formed of a compressible material that allows the proximal portion to move axially relative to the distal portion. The body cavity, proximal channel, and distal channel form a fluid tight cavity when the proximal portion is engaged with the shaft of the delivery system and the distal portion is engaged with a capsule.

In another aspect, the present disclosure provides a transfer sleeve for use with a delivery system for an implantable medical device. The transfer sleeve includes a proximal portion defining a proximal channel. A portion of the proximal portion extends from a central axis of the proximal portion to engage with an edge of an introducer sheath when the delivery system is inserted into the introducer sheath. The proximal channel has a cross-sectional area that allows a shaft of the delivery system to fit within the proximal channel of the proximal portion. The transfer sleeve also includes a distal portion defining a distal channel, wherein the distal channel has a cross-section area that allows the shaft of the delivery system to fit within the distal channel of the distal portion. Further, the transfer sleeve includes a body portion coupled between the proximal portion and the distal portion and defining a body channel. The distal portion and the body portion have exterior diameters that enable the distal portion and the body portion to enter a lumen of the introducer sheath. The body channel, proximal channel, and distal channel form a fluid tight cavity when the proximal portion is engaged with the shaft of the delivery system and the distal portion is engaged with a capsule.

In an example, the present disclosure provides a method (not claimed). The method includes installing a transfer sleeve onto a catheter portion of a delivery system. The transfer sleeve creates a fluid tight cavity surrounding an exposed portion of an implantable medical device coupled to the catheter portion of the delivery system. The method also includes inserting the catheter portion of the delivery system into an introducer sheath, the introducer sheath providing access to a vessel. Further, the method includes advancing the catheter portion through the introducer sheath. The transfer sleeve contacts a surface of the introducer sheath as the catheter portion advances through the introducer sheath. The contact causes the transfer sleeve to remain stationary relative to the catheter portion as the catheter portion advances through the introducer sheath.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

The foregoing and other features and advantages of the present disclosure will be apparent from the following description of embodiments hereof as illustrated in the accompanying drawings. The accompanying drawings, which are incorporated herein and form a part of the specification, further serve to explain the principles of the present disclosure and to enable a person skilled in the pertinent art to make and use the embodiments of the present disclosure. The drawings are not to scale.
FIGS. 1A-1C depict several illustrations of a delivery system that may be used with transfer sleeves, according to an embodiment hereof.
FIGS. 2A-2E depict several illustrations of a transfer sleeve for use with a delivery system, according to an embodiment hereof.
FIGS. 3A-3D depict several illustrations of a transfer sleeve for use with a delivery system, according to another embodiment hereof.
FIG. 4 depicts a method for using a transfer sleeve with a delivery system, according to an embodiment hereof.
FIGS. 5A-5E depict several illustrations of the method of FIG. 4 performed using the transfer sleeves of FIGS. 2A-2E or FIGS. 3A-3D, according to an embodiment hereof.
FIGS. 6A-6E depict several illustrations of a transfer sleeve for use with a delivery system, according to another embodiment hereof.
FIGS. 7A-7I depict several illustrations of the method of FIG. 4 performed using the transfer sleeve of FIGS. 6A-6E, according to an embodiment hereof.
FIG. 8 depicts an illustration of a prosthetic heart valve that may be used with the delivery systems and transfer sleeves of the embodiments hereof.

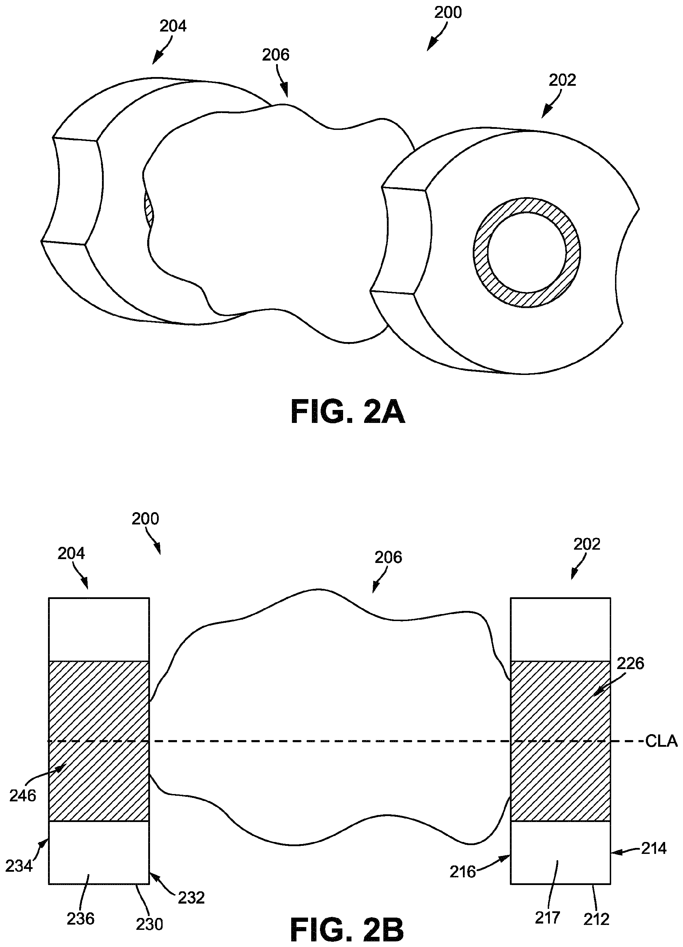

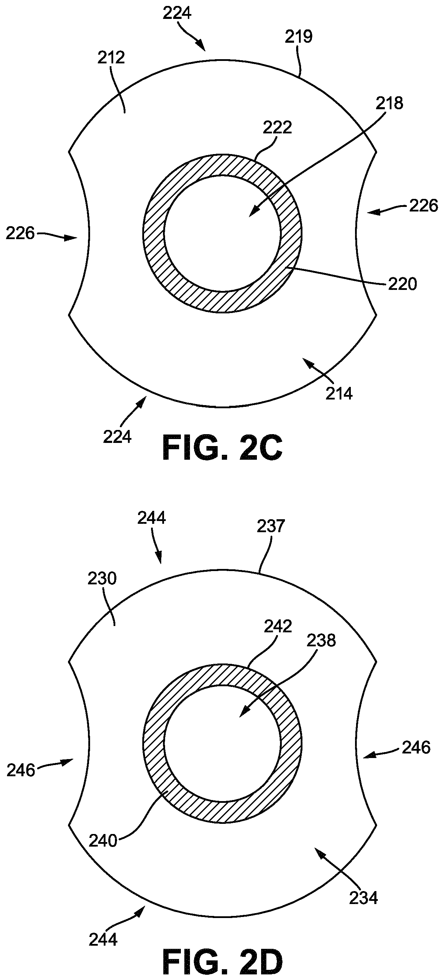

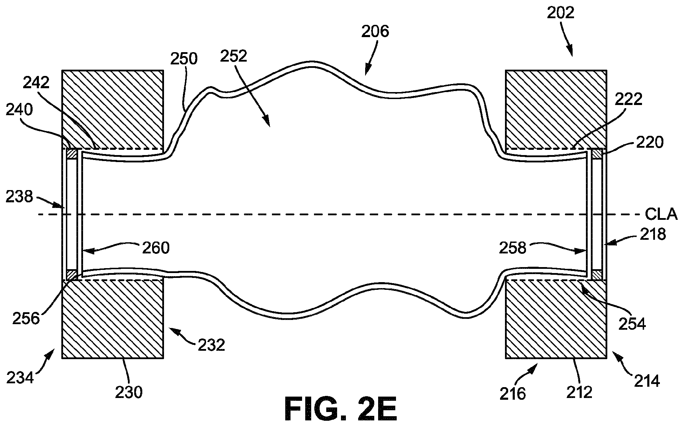

The invention relates to a transfer sleeve as illustrated in fig. 2-3, 5.

### DETAILED DESCRIPTION

Specific embodiments of the present disclosure are now described with reference to the figures. The following detailed description describes examples of embodiments and is not intended to limit the present technology or the application and uses of the present technology. Although the description of embodiments hereof is in the context of a delivery system that may be used with an implantable medical device, the present technology may also be used in other devices. Furthermore, there is no intention to be bound by any expressed or implied theory presented in the preceding technical field, background, brief summary or the following detailed description.

The terms "distal" and "proximal", when used in the following description to refer to a delivery system or catheter are with respect to a position or direction relative to the treating clinician. Thus, "distal" and "distally" refer to positions distant from, or in a direction away from the treating clinician, and the terms "proximal" and "proximally" refer to positions near, or in a direction toward the clinician.

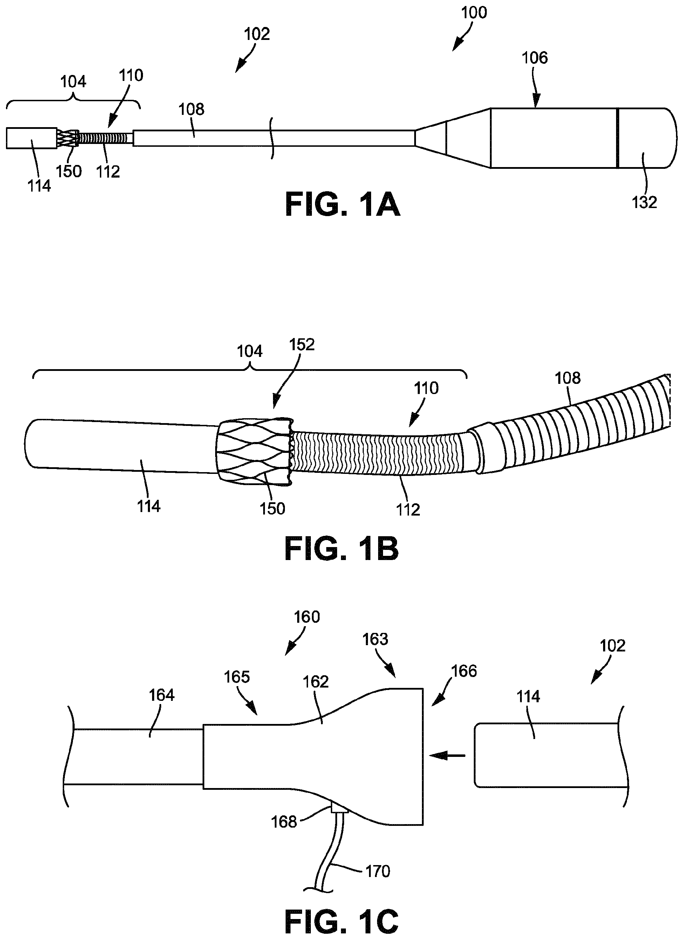

Embodiments disclosed herein are directed to transfer sleeves for a delivery system for implantable medical devices. In embodiments, the transfer sleeves are configured to provide a sterile environment for an implantable medical device after installation on a delivery system prior to introduction into a patient. In embodiments described herein, the transfer sleeves are configured to operate in combination with a delivery system that operates to deliver an implantable medical device to an implant location. FIGS. 1A-1C illustrate an example of a delivery system 100 in accordance with an embodiment hereof. One skilled in the art will realize that FIGS. 1A-1C illustrate one example of a delivery system and that existing components illustrated in FIGS. 1A-1C may be removed and/or additional components may be added to the delivery system 100.

As shown in FIG. 1A, the delivery system 100 generally comprises a catheter portion 102 having a distal portion 104. The catheter portion 102 is coupled to a control handle portion 106 by which the catheter portion 102 is manipulated to deliver an implantable medical device 150, *e.g.,* a prosthetic heart valve including a prosthetic valve structure and a stent, to an implant location and to deploy the implantable medical device 150 at the implant location. The catheter portion 102 is preferably of a length and size so as to permit a controlled delivery of the distal portion 104 to the implant location, *e.g.,* a patient's heart. In embodiments, the catheter portion 102 includes features to enhance maneuverability, steerability and advancement of the distal portion 104 to the implant location. As discussed in further detail below, the distal portion 104 provides the means by which an implantable medical device 150 can be mounted for delivery to the implant location and further enables the expansion of the implantable medical device 150 for effective deployment thereof. The control handle portion 106 preferably controls movements as translated to the distal portion 104 by way of elongate structure of the catheter portion 102. Controlled functionality from the control handle portion 106 is preferably provided in order to permit expansion and deployment of the implantable medical device 150 at a desired location, such as a heart valve annulus, and to provide for ease in the delivery and withdrawal of the delivery system through a patient's vasculature.

The catheter portion 102 of the delivery system 100 also preferably comprises an outer shaft 108 that is also operatively connected with the control handle portion 106 and that surrounds one or more inner shafts, such as an inner shaft 110, over at least a part of its length. The inner shaft 110 may include a flexible portion 112 that allows maneuverability, steerability and/or advancement of the distal portion 104 to the implant location. In embodiments, the outer shaft 108 comprises a lubricous inner layer (such as high density polyethylene HDPE or Polytetrafluoroethylene PTFE), and/or a braided stainless steel middle layer and/or with a flexible plastic outer layer, such as comprised of Pebax 7233, or Nylon 12. The outer shaft 108 extends from the control handle portion 106 and facilitates the advancement and steering of the delivery system through a patient's vasculature by improving the pushability of the delivery system 100.

The inner shaft 110 may be operatively connected with the control handle portion 106 so as to be movable by operation of the control handle portion 106. As illustrated in FIG. 1B, which is an enlarged view of the distal portion 104, an implantable medical device 150 may be coupled to the inner shaft 110 in a compressed (non-expanded) state for delivery to the implant location. A capsule 114 may be removably placed over a portion of the implantable medical device 150. The capsule 114 operates to protect the implantable medical device 150 during delivery to the implant location through a patient's vasculature. In embodiments, the capsule 114 can be reduced in length, *e.g*., axial length, to allow for tracking and deployment of the implantable medical device 150. For example, during a transseptal mitral valve replacement procedure, the inner shaft 110 can be bent at the flexible portion 112 to steer the implantable medical device 150 (*e.g.,* a mitral prosthetic heart valve) through chambers of a patient's heart. For example, the control handle portion 106 can include an adjustable handle control 132 that can be manipulated, *e.g*., rotated, to deflect the flexible portion 112 of the inner shaft 110. As such, the capsule 114 can be limited in length to prevent interference with the flexible portion 112 of the inner shaft 110 when installed over the implantable medical device 150. Due to the reduced length of the capsule 114, a portion 152 of the implantable medical device 150 may not contained within the capsule 114*, e.g.,* a brim of the implantable medical device 150.

Once the implantable medical device 150 is positioned at the implant location, the capsule 114 can be removed from the implantable medical device 150, and the implantable medical device 150 can be transitioned from a compressed state to an uncompressed (expanded) state to engage native anatomy at the implant location. For example, the implantable medical device 150 can be loaded over a shaft assembly (not shown) that is coupled to the inner shaft 110. The implantable medical device 150 can be compressively retained within the capsule 114. The shaft assembly can include a retention member (*e.g*., spindle) which is configured to selectively receive corresponding features of the implantable medical device 150 (*e.g.,* paddles, posts, or eyelets). In some embodiments, as shown, the capsule 114 can be removed distally by utilizing an additional catheter or device. In some embodiments, the capsule 114 can be removed proximally, for example, by attachment to a member of the catheter portion 102, which can be withdrawn.

In some embodiments, the implantable medical device 150 can be self-expanding. For example, the implantable medical device 150 can be constructed of a material that transitions from the compressed state to the uncompressed state when the capsule 114 is removed and the implantable medical device 150 is decoupled from the inner shaft 110. For example, a stent or frame of the implantable medical device can be formed from a shape memory material such as a nickel titanium alloy (*e.g.,* Nitinol) that is self-expandable from the compressed state to the expanded state, such as by the application of heat, energy, and the like, or by the removal of external forces (*e.g*., compressive forces). In some embodiments, the implantable medical device 150 can be expanded using expansion devices such as a balloon.

In order to deliver the implantable medical device 150 to the implant location, *e.g.,* track a prosthetic heart valve to a patient's heart, a clinician first establishes percutaneous access to a patient's vasculature. In embodiments, as illustrated in FIG. 1C, an introducer sheath 160 can be utilized in combination with the delivery system 100 for establishing access to the patient's vasculature. As illustrated in FIG. 1C, the introducer sheath 160 may include a sheath portion 164 and/or a handle portion 162 attached at a proximal end of the sheath portion 164. The sheath potion 164 can be a tubular structure that defines a central or interior lumen from the proximal end to a distal end (not shown) of the sheath portion 164. The handle portion 162 can include a distal portion 165 coupled to the proximal end of the sheath portion 164 and/or a proximal portion 163 that has an enlarged diameter relative to the distal portion 165. The handle portion 162 can define a central or interior lumen that can decrease, *e.g*., taper, in diameter from the proximal portion 163 to the distal portion 165, where the interior lumen of the handle portion 162 couples to the interior lumen of the sheath portion 164 at the connection of the handle portion 162 and the sheath portion 164. The handle portion 162 defines an entry port 166 in which the catheter portion 102 of the delivery system 100 can be inserted. In some embodiments, the handle portion 162 can include a flush tube 170 coupled to the interior lumen of the handle portion 162 by a port 168.

In embodiments, in a method of providing percutaneous access to a patient's vasculature, the introducer sheath 160 can be inserted into a vessel, *e.g.,* a femoral, brachial, or radial artery, using a puncture needle (not shown) inserted through the skin and into the vessel. Once the puncture needle is inserted into the vessel, the introducer sheath can be advanced over the puncture needle and a dilator (not shown) until the distal end of the sheath portion 164 of the introducer sheath 160 enters the vessel thereby providing access to the vessel. Once inserted into the vessel and the puncture needle and dilator are removed, the introducer sheath 160 can hold a tract open and protect the vessel from trauma as the catheter portion 102 of the delivery system 100 is introduced into the vessel therethrough. That is, the distal portion 104 of the catheter portion 102 can be inserted into the entry port 166 through the lumens of the handle portion 162 and the sheath portion 164, thereby entering the vessel.

In embodiments, the implantable medical device 150 needs to be protected and maintained in a sterile environment after being loaded onto the catheter portion 102 and prior to the catheter portion 102 being inserted through the introducer sheath 160 into a patient's vasculature. To achieve this, a transfer sleeve can be utilized to protect the implantable medical device 150 during the transfer and introduction to a patient's body through the introducer sheath 160.

FIGS. 2A-2E illustrate an example of a transfer sleeve 200 in accordance with an embodiment hereof. One skilled in the art will realize that FIGS. 2A-2E illustrate one example of a transfer sleeve and that existing components illustrated in FIGS. 2A-2E may be removed and/or additional components may be added to the transfer sleeve 200.

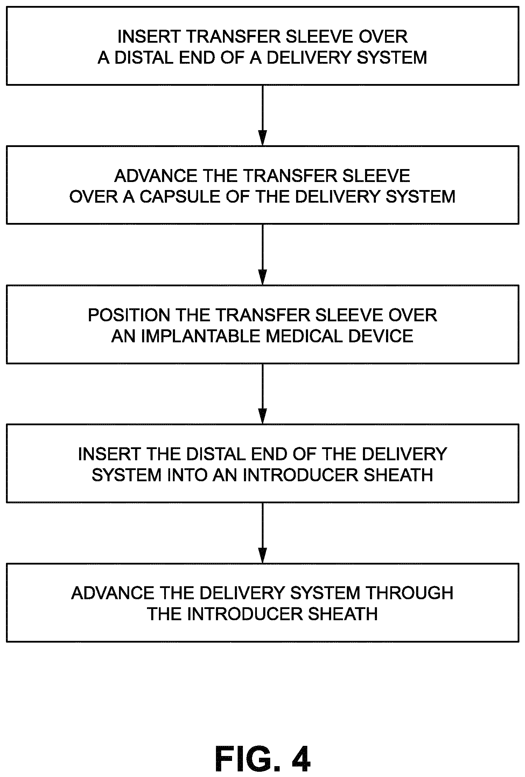

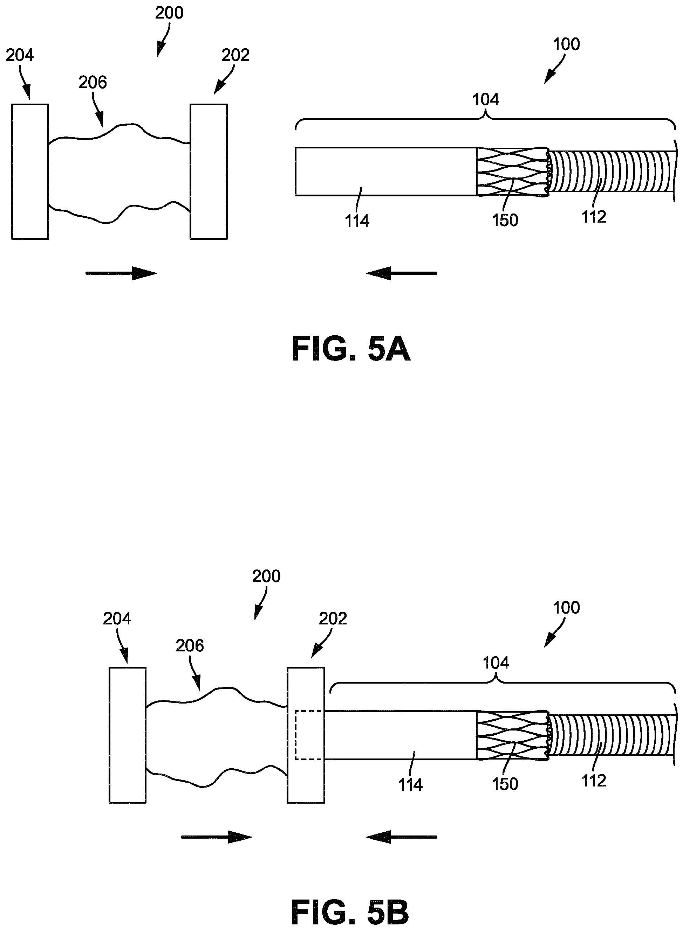

As illustrated in FIG. 2A, which is a perspective view of the transfer sleeve 200, the transfer sleeve 200 includes a proximal portion 202 and a distal portion 204. As illustrated in FIG. 2A and FIG. 2B, which is a side view of the transfer sleeve 200, a body portion 206 is coupled between the proximal portion 202 and the distal portion 204. The transfer sleeve 200 is configured to be positioned over a distal portion of a delivery device, *e.g.,* the distal portion 104 of the delivery system 100. For example, the transfer sleeve 200 can be installed over the implantable medical device 150 that is installed on the delivery system 100, as described in further detail below with reference FIGS. 4 and 5A-5D. When installed, the proximal portion 202 is positioned to be on the proximal side of the implantable medical device 150, and the distal portion 204 is positioned on the distal side of the implantable medical device 150 such that the implantable medical device 150 is located within the body portion 206. The proximal portion 202 may seal to a first portion of the delivery system, *e.g*., the inner shaft 110, and/or the distal portion 204 may seal to a second portion of the delivery system, *e.g*., the capsule 114, thereby providing a sterile environment for implantable medical device 150.

The proximal portion 202 may include a seal clamp 212 that includes a proximal side 214, a distal side 216, and a body 217 extending from the proximal side 214 to the distal side 216. As illustrated in FIG. 2C, which is a view of the proximal side 214, the seal clamp 212 may be formed having a solid body that includes a channel 218 that extends from the proximal side 214 to the distal side 216 of the seal clamp 212. An outer surface 219 of the body 217 can be formed having one or more curved portions 224, *e.g.,* two curved portions 224, extending between one or more indentations 226, *e.g.,* two indentations 226. In some embodiments, the two indentations 226 can provide a surface for gripping the seal clamp 212. While FIG. 2C illustrates the seal clamp 212 including the indentations 226, in some embodiments, the outer surface 219 of the seal clamp 212 can be formed having a circular cross-section thereby defining a cylindrical shape for the seal clamp 212. The seal clamp 212 can be formed of a rigid or semi-rigid material, *e.g*., silicon, rubber, latex, polymeric material, *etc.*

In embodiments, the channel 218 can be formed having a circular cross-section, thereby forming a channel from the proximal side 214 to the distal side 216 having a cylindrical shape. A radial seal 220 may be positioned inside the channel 218. The radial seal 220 may be coupled to an inner surface 222 of the body 217 the seal clamp 212 defining the channel 218. The radial seal 220 may extend inward from the inner surface 222 towards a central longitudinal axis ("CLA") of the seal clamp 212. The radial seal 220 is configured to form a seal with components of a delivery system when the transfer sleeve 200 is installed on the delivery system. The radial seal 220 can be formed of a flexible material, *e.g*., latex or other polymeric flexible material, that expands, contracts, and moves when components of a delivery system are inserted through the channel 218.

The distal portion 204 may include a seal clamp 230 that includes a proximal side 232, a distal side 234, and a body 236 extending from the proximal side 232 to the distal side 234. As illustrated in FIG. 2D, which is a view of the distal side 234, the seal clamp 230 may be formed having a solid body that includes a channel 238 that extends from the proximal side 232 to the distal side 234 of the seal clamp 230. An outer surface 237 of the body 236 can be formed having one or more curved portions 244, *e.g.,* two curved portions 244, extending between one or more indentations 246, *e.g.,* two indentations 246. In some embodiments, the two indentations 246 can provide a surface for gripping the seal clamp 230. While FIG. 2D illustrates the seal clamp 230 including the indentations 246, in some embodiments, the outer surface 237 of the seal clamp 230 can be formed having a circular cross-section thereby defining a cylindrical surface.

In embodiments, the channel 238 can be formed having a circular cross-section, thereby forming a channel from the proximal side 232 to the distal side 234 having a cylindrical shape. A radial seal 240 may be positioned inside the channel 238. The radial seal 240 may be coupled to an inner surface 242 of the body 236 defining the channel 238. The radial seal 240 may extend inward from the inner surface 242 towards a central longitudinal axis, CLA, of the seal clamp 230. The radial seal 240 is configured to form a seal with components of a delivery system when the transfer sleeve 200 is installed on the delivery system. The radial seal 240 can be formed of a flexible material, *e.g*., latex or other polymeric flexible material, that expands, contracts, and moves when components of a delivery system are inserted through the channel 238.

In some embodiments, the seal clamp 212 and the seal clamp 230 can be constructed having different dimensions. In some embodiments, the seal clamp 212 and the seal clamp 230 can be constructed having similar dimensions. In any embodiment, the seal clamp 212 can be construed having an outer diameter, *e.g*., diameters of the proximal surface 214 and the distal surface 216 to the curved portions 224, that enables the seal clamp 212 to engage, directly or indirectly, with components of an introducer sheath, *e.g.,* the handle portion 162 of the introducer sheath 160. Likewise, the seal clamp 230 can be construed having an outer diameter, *e.g*., diameters of the proximal surface 232 and the distal surface 234 to the curved portions 244, that enables the seal clamp 230 to engage, directly or indirectly, with components of an introducer sheath, *e.g.,* the handle portion 162 of the introducer sheath 160. As such, as a catheter portion 102 is inserted through the introducer sheath 160, the handle portion 162 applies a force on the transfer sleeve 200 thereby causing the transfer sleeve 200 to move in a proximal direction relative to the catheter portion 102, *e.g.,* the catheter portion 102 moves through the sheath portion 164 as the transfer sleeve 200 remains in the handle portion 162, as described below in further detail.

In any embodiment, the channel 218 and the channel 238 can be constructed having diameters that allow components of a delivery system, *e.g*., the outer shaft 108, the inner shaft 110, and the capsule 114 of the delivery system 100, to pass through the channel 218 and the channel 238, while still engaging with the radial seal 220 and the radial seal 240. For example, an outer diameter of the seal clamp 212 (or the seal clamp 230) can be approximately 40 millimeters (mm). Likewise, for example, the diameter of the channel 218 (or the channel 238) can be approximately 15 mm, and the inner diameter of the radial seal 220 (or radial seal 240) can be approximately 7 mm. One skilled in the art will realize that any examples of dimensions describe herein are approximate values and can vary by, for example, +/- 5.0 %, based on manufacturing tolerances, operating conditions, and/or other factors.

As illustrated in FIG. 2E, which is a cross-sectional view of the transfer sleeve 200, the body portion 206 can be formed of a single piece material 250 that forms a cavity 252. The single piece material 250 includes a proximal end 254 and a distal end 256. The proximal end 254 includes a proximal opening 258. In some embodiments, the proximal end 254 of the single piece material 250 can be coupled to the distal surface 216 of the seal clamp 212. In some embodiments, as illustrated in FIG. 2E, the proximal end 254 of the single piece material 250 extends into the channel 218 of the seal clamp 212 from the distal side 216. The proximal end 254 of the single piece material 250 can be coupled to the inner surface 222 of the body 217 defining the channel 218. In some embodiments, the radial seal 220 can be formed as an integral part of the single piece material 250.

The distal end 256 includes a distal opening 260. In some embodiments, the distal end 256 of the single piece material 250 can be coupled to the proximal surface 232 of the seal clamp 230. In some embodiments, as illustrated in FIG. 2E, the distal end 256 of the single piece material 250 extends into the channel 238 of the seal clamp 230 from the proximal side 232. The distal end 256 of the single piece material 250 can be coupled to the inner surface 242 of the body 235 defining the channel 238. In some embodiments, the radial seal 240 can be formed as an integral part of the single piece material 250.

In embodiments, the channel 218 of the seal clamp 212 and the channel 238 of the seal clamp 230 are in fluid communication with the cavity 252. When the radial seal 220 of the seal clamp 212 and the radial seal 242 of the seal clamp 230 engage with components of the delivery system 100, the cavity 252 forms a fluid tight chamber in which exposed portions of the implantable medical device 150 can be contained. The single piece material 250 can be any type of flexible material that can be compressed when the distal portion 204 moved towards the proximal portion 202 or vice versa. In some embodiments, the single piece material 250 can be formed of flexible material, *e.g.*, latex or other polymeric flexible material. In some embodiments, the single piece material 250 can be transparent or semi-transparent thereby providing a view or partial view of the cavity 252. In embodiments, the cavity 252 can be constructed to a length that allow the seal clamp 212 and the seal clamp 230 to engage with components of the delivery system 100 on either side of an exposed portion 152 of the implantable medical device thereby containing the exposed portion 152 with in the cavity 252, when the single piece material is in an expanded or partially expanded state. For example, a length of the cavity 252 can be approximately 40 mm.

Due to the flexibility of the single piece material 250, the body portion 206 can move, flex, and compress as the components of the delivery system 150 move during insertion through the introducer sheath 160. For example, as the catheter portion 102 is inserted through the introducer sheath 160, the handle portion 162 applies a force on the seal clamp 230 (or the seal clamp 212 depending on an installed orientation of the transfer sleeve 200) that causes the seal clamp 230 to move in a proximal direction relative to the catheter portion 102 thereby compressing the single piece material 250 until the seal clamp 230 abuts the seal clamp 212 (or vice versa). Once the seal clamp 230 abuts the seal clamp 212, the seal clamp 230 applies a force on the seal clamp 212 thereby causing the transfer sleeve 200 to move in a proximal direction relative to the catheter portion 102 of the delivery system 100.

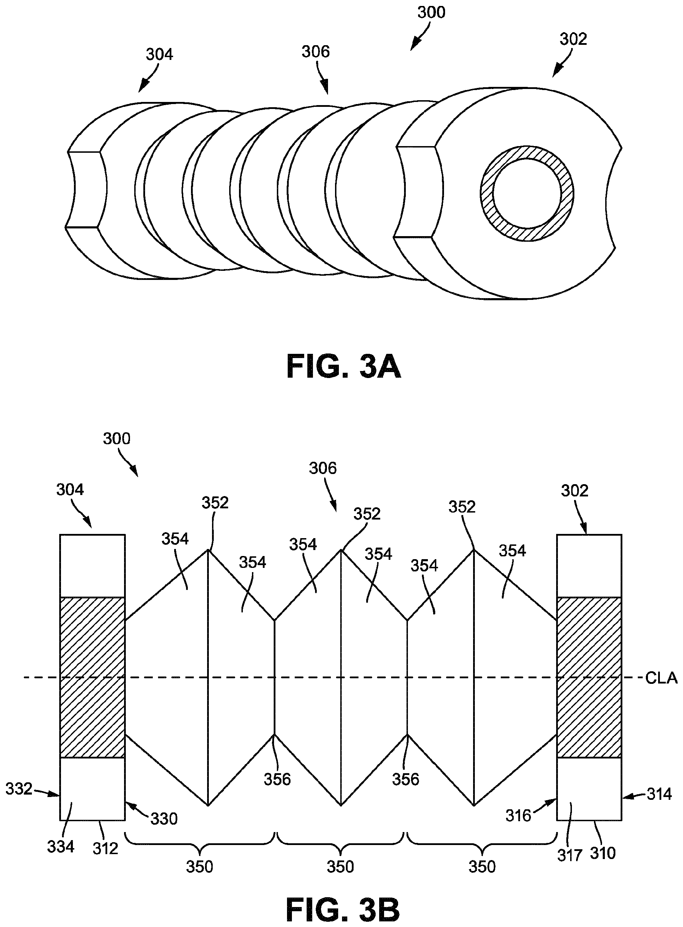

FIGS. 3A-3D illustrate another example of a transfer sleeve 300 in accordance with an embodiment hereof. One skilled in the art will realize that FIGS. 3A-3D illustrate one example of a transfer sleeve and that existing components illustrated in FIGS. 3A-3D may be removed and/or additional components may be added to the transfer sleeve 300.

As illustrated in FIG. 3A, which is a perspective view of the transfer sleeve 300, the transfer sleeve 300 includes a proximal portion 302 and a distal portion 304. As illustrated in FIG. 3A and FIG. 3B, which is a side view of the transfer sleeve 300, a body portion 306 is coupled between the proximal portion 302 and the distal portion 304. Similar to the transfer sleeve 200, the transfer sleeve 300 is configured to be positioned over a distal portion of a delivery device, *e.g.,* the distal portion 104 of the delivery system 100, to protect and to provide a sterile environment for an implantable medical device installed thereon.

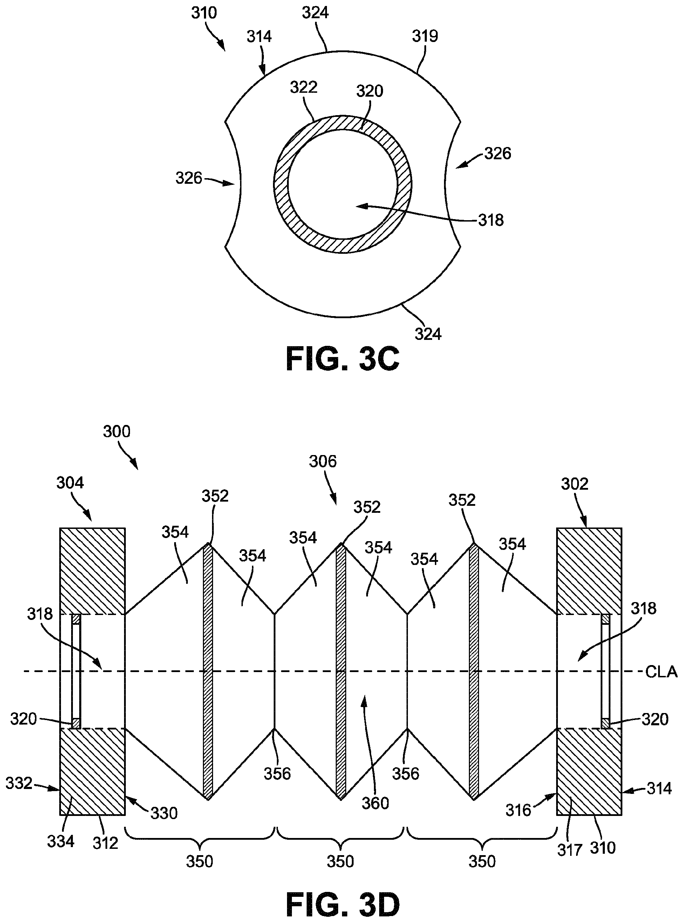

The proximal portion 302 may include a seal clamp 310 that includes a proximal side 314, a distal side 316, and a body 317 extending from the proximal side 314 to the distal side 316. As illustrated in FIG. 3C, which is a view of the proximal side 314, the seal clamp 310 may be formed having a solid body that includes a channel 318 that extends from the proximal side 314 to the distal side 316 of the seal clamp 310. An outer surface 319 of the seal clamp 310 can be formed having one or more curved portions 324, *e.g.,* two curved portions 324, extending between one or more indentations 326, *e.g.,* two indentations 326. In some embodiments, the two indentations 326 can provide a surface for gripping the seal clamp 312. While FIG. 3C illustrates the seal clamp 310 including the indentations 326, in some embodiments, the outer surface 319 of the seal clamp 310 can be formed having a circular cross-section thereby defining the seal clamp 310 having a cylindrical shape.

In embodiments, the channel 318 can be formed having a circular cross-section, thereby forming a channel from the proximal side 314 to the distal side 316 having a cylindrical shape. A radial seal 320 may be positioned inside the channel 318. The radial seal 320 may be coupled to an inner surface 322 of the body 217 defining the channel 318. The radial seal 320 may extend inward from the inner surface 322 towards a central longitudinal axis, CLA, of the seal clamp 310. The radial seal 320 is configured to form a seal with components of a delivery system when the transfer sleeve 300 is installed on the delivery system. The radial seal 320 can be formed of a flexible material, *e.g*., latex or other polymeric flexible material, that expands, contracts, and moves when components of a delivery system are inserted through the channel 318.

As illustrated in FIG. 3B, the distal portion 304 may include a seal clamp 312 that includes a proximal side 330, a distal side 332, and a body 334 extending from the proximal side 332 to the distal side 334. While not illustrated, in some embodiments, the seal clamp 312 can be configured and include the same components as the seal clamp 310. That is, the seal clamp 312 may be formed having a solid body that includes a channel that extends from the proximal side 330 to the distal side 332 of the seal clamp 312. Similar to seal clamp 310, an outer surface of the seal clamp 312 can be formed having one or more curved portions, *e.g*., two curved portions, extending between one or more indentations, *e.g.,* two indentations.

In some embodiments, the channel formed in the seal clamp 312 from the proximal side 330 to the distal side 332 can be formed having the same configuration (*e.g*., dimensions, shape, *etc.*) as the channel 318. Likewise, similar to seal clamp 310, the seal clamp 312 can include a radial seal that is positioned inside the channel the proximal side 330 to the distal side 332 and is coupled to an inner surface of the body 334 defining the channel. The radial seal can be formed having the same configuration (*e*.*g*., dimensions, shape, *etc.*) as the radial seal 320.

In any embodiment, the seal clamp 310 (and similarly the seal clamp 312) can be construed having an outer diameter, *e.g*., diameters of the proximal surface 314 and the distal surface 316 to the curved portions 324, that enables the seal clamp 310 to engage, directly or indirectly, with components of an introducer sheath, *e.g.,* the handle portion 162 of the introducer sheath 160. As such, as a catheter portion 102 is inserted through the introducer sheath 160, the handle portion 162 applies a force on the transfer sleeve 300 thereby causing the transfer sleeve 300 to move in a proximal direction relative to the catheter portion 102, *e.g.,* the catheter portion 102 moves through the sheath portion 164 as the transfer sleeve 300 remains in the handle portion 162, as described below in further detail.

In any embodiment, the channel 318 of the seal clamp 310 (and the channel of the seal clamp 312) can be constructed having diameters that allows components of a delivery system, *e.g*., the outer shaft 108, the inner shaft 110, and the capsule 114 of the delivery system 100, to pass through the channel 318 of the seal clamp 310 (and the channel of the seal clamp 312), while still engaging with the radial seal 320 (and the radial seal of the seal clamp 312). For example, an outer diameter of the seal claim 310 (or the seal clamp 312 can be approximately 40 mm. Additionally, for example, a diameter of the channel 318 can be approximately 15 mm. Further, for example, an inner diameter of the radial seal 320 can be approximately 7 mm.

As illustrated in FIG. 3B and FIG. 3D, which is a cross-sectional view of the transfer sleeve 300, the body portion 306 may include flexible sections 350, in particular such that the body portion 306 moves and compresses like an accordion. The flexible sections 350 are positioned along a central longitudinal axis, CLA, from the proximal portion 302 to the distal portion 304. The flexible sections 350 are configured to flex, compress, and/or expand as the proximal portion 302 moves relative to the distal portion 304. That is, the flexible sections 350 enable the body portion 306 to move with an accordion-like motion.

Each of the flexible sections 350 may include a circular rib 352 and/or a pair of flexible membranes 354. Adjacent flexible sections 350 may be coupled at a circular rib 356. That is, the flexible membranes 354 of adjacent flexible sections 350 are coupled to a circular rib 356. The flexible section 350 may form a cavity 360. The circular ribs 352 and the circular ribs 356 can be construed of a rigid or semi-rigid material, *e.g*., silicon, rubber, latex, polymeric material, *etc.* The flexible membranes 354 can be construed of a flexible material, *e.g.,* silicon, rubber, latex, polymeric material, *etc.* Due to the flexibility of the flexible membranes 354, the flexible sections 350 can move, flex, and compress as the components of the delivery system move during insertion into an introducer sheath. For example, as a catheter portion 102 is inserted through the introducer sheath 160, the handle portion 162 applies a force on the seal clamp 312 (or the seal clamp 310 depending on an installed orientation of the transfer sleeve 300) that causes the seal clamp 312 to move in a proximal direction relative to the catheter portion 102 thereby compressing the flexible sections 350 until the seal clamp 312 abuts the seal clamp 310 (or vice versa). Once the seal clamp 312 abuts the seal clamp 310 (the flexible section 350 being fully compressed), the seal clamp 312 applies a force on the seal clamp 310 thereby causing the transfer sleeve 300 to move in a proximal direction on the catheter portion 102 of the delivery system 100.

In embodiments, the cavity 360 can be constructed to a length that allow the seal clamp 310 and the seal clamp 312 to engage with components of the delivery system 100 on either side of an exposed portion 152 of the implantable medical device thereby containing the exposed portion 152 with in the cavity 360, when the body portion 306 is in an expanded or partially expanded state. For example, a length of the cavity 252 can be approximately 40 mm. Additionally, a diameter of the circular ribs 350 can be approximately 15 mm, and a diameter of the circular ribs 356 can be approximately 35 mm.

FIGS. 4 and 5A-5D illustrate an example of a method 400 utilizing a transfer sleeve, for example, transfer sleeve 200 or transfer sleeve 300, in accordance with an embodiment hereof. One skilled in the art will realize that FIGS. 4 and 5A-5D illustrate one example of a method using a transfer sleeve and that operations illustrated in FIG. 4 may be removed and/or additional operations may be added to the method 400. One skilled in the art will also realize that the installation of the transfer sleeve 200 or transfer sleeve 300 onto the delivery system can be performed while portions of the delivery system containing the implantable medical device are submerged in a sterile bath.

In step 402, a transfer sleeve can be inserted over a distal end of a delivery system. For example, as illustrated in FIG. 5A, which illustrates the method 400 being performed using the transfer sleeve 200 and the delivery system 100, the transfer sleeve 200 can be positioned so that the proximal portion 202 is facing the distal end (the capsule 114) of the catheter portion 102 (the distal portion 104). Once positioned, the transfer sleeve 200 and the distal portion 104 can be advanced towards one another, e.g., the transfer sleeve 200 is advanced towards the capsule 114 and/or the capsule 114 is advanced towards the proximal portion 202. As illustrated in FIG. 5B, the capsule 114 of the distal portion 104 can be inserted into the proximal portion 202 of the transfer sleeve 200. That is, the capsule 114 of the catheter portion 102 can be inserted into the channel 218 of the seal clamp 212. As the capsule 114 enters channel 218, the radial seal 220 engages with the outer surfaces of the capsule 114 creating a fluid tight seal between the capsule 114 and the seal clamp 212.

In step 404, the transfer sleeve can be advanced over a capsule of the delivery system. For example, as illustrated in FIG. 5B, the transfer sleeve 200 and the catheter portion 102 of the delivery system 100 continue to be advanced towards one another until the capsule 114 passes into the body portion 206 of the transfer sleeve 200. Then, the transfer sleeve 200 and the catheter portion 102 of the delivery system 100 continue to be advanced towards one another until the capsule 114 enters the channel 238 of the seal clamp 230.

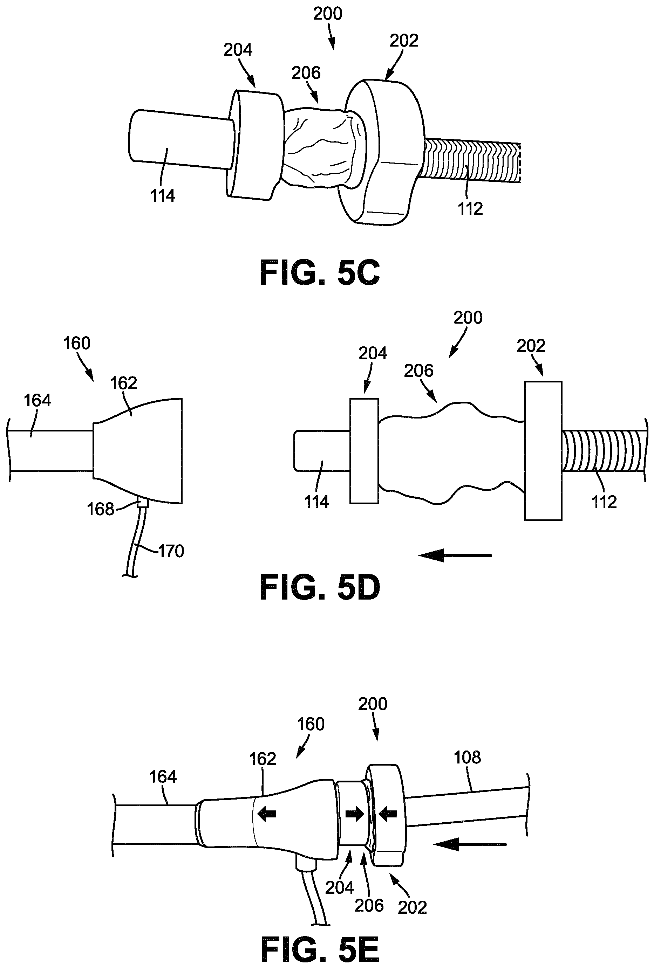

In step 406, the transfer sleeve can be position over the implantable medical device. For example, the advancement of the transfer sleeve 200 is continued until the seal clamp 212 is positioned on the inner shaft 110 proximal of the implantable medical device 150 and the capsule 114 extends from the seal clamp 230, thereby positioning any exposed portion of the implantable medical device 150 within the cavity 252 of the body portion 206, as illustrated in FIG. 5C. Because the cavity 252 is fluid tight, sterile fluid utilized during the installation of the transfer sleeve 200, e.g., sterile fluid bath, is contained within the cavity 252 thereby providing a sterile environment for any exposed portion of the implantable medical device 150.

In step 408, the distal end of the delivery system can be inserted into an introducer sheath. For example, once the implantable medical device 150 is ready for delivery to an implant site, the distal end 104 of the catheter portion 102 can be inserted into the introducer sheath 160 to introduce the catheter portion 102 to a patient's vasculature. While not shown, prior to insertion of the catheter portion 102, the introducer sheath 160 can be installed in a vessel of a patient using known techniques, *e.g*., puncture needle and dilator. As illustrated in FIG. 5D, the distal end 104 of delivery system 100 (including the transfer sleeve 200) is advanced towards the handle portion 162 of the introducer sheath 160. The distal end 104 of the delivery system 100 is advanced until the capsule 114 enters the central lumen of the handle portion 162.

In step 410, the delivery system can be advanced through the introducer sheath. The distal end 104 of the delivery system 100 is advanced so that the capsule 114 enters the central lumen of the sheath portion 164 of the introducer sheath 160. As the capsule 114 enter the central lumen of the handle portion 162 and is advanced, the distal portion 204 (seal clamp 230) of the transfer sleeve 200 abuts the handle portion 162 of the introducer sheath 160. As the catheter portion 102 is advanced through the introducer sheath 160, the handle portion 162 applies a force on the distal portion 204 (seal clamp 230) that prevents the distal portion 204 (seal clamp 230) from moving distally with the catheter portion 102, thereby forcing the distal portion 204 (seal clamp 230) to slide along the catheter portion 102 such that distal portion 204 (seal clamp 230) is disposed more proximally on the catheter portion 102. Meanwhile, the proximal portion 202 (seal clamp 212) moves distally with the catheter portion 102, thereby compressing the body portion 206 (single piece material 250) until the distal portion 204 (seal clamp 230) abuts the proximal portion 202 (seal clamp 212). Once the distal portion 204 (seal clamp 230) abuts the proximal portion 202 (seal clamp 212), the force on the proximal portion 202 (seal clamp 212) prevents the proximal portion 202 (seal clamp 212) from moving distally with the catheter portion 102, thereby forcing the proximal portion 202 (seal clamp 212) to slide along the catheter portion 102 with the distal portion 204 (seal clamp 230) so that both are disposed more proximally on the catheter portion 102, as illustrated in FIG. 5E.

As the transfer sleeve 200 moves proximally on the inner shaft 110 due to the catheter portion 102 being advanced through the introducer sheath, the implantable medical device 150 passes out of the transfer sleeve 200 into the introducer sheath 160. Because the transfer sleeve 200 abuts the handle portion 162, the implantable medical device 150 is only exposed to the central lumens of the introducer sheath 160, thereby maintaining a sterile environment for the implantable medical device 150. In some embodiments, the transfer sleeve 200 can remain on the inner shaft 110 of the catheter portion 102 and advance proximally as the inner shaft 110 is advanced through the introducer sheath 160. In some embodiments, the transfer sleeve 200 can be advanced proximally until the transfer sleeve 200 is positioned on the outer shaft 108 of the catheter portion 102.

While the method 400 is described above with the transfer sleeve 200 being oriented such that the proximal portion 202 engages first with the distal portion 104 of the delivery system 100, the method 400 can also be performed such that the distal portion 204 engages first with the distal portion 104 of the delivery system 100. Additionally, while the method 400 is described above with reference to the transfer sleeve 200, the method 400 (and any modifications) can also be performed using the transfer sleeve 300.

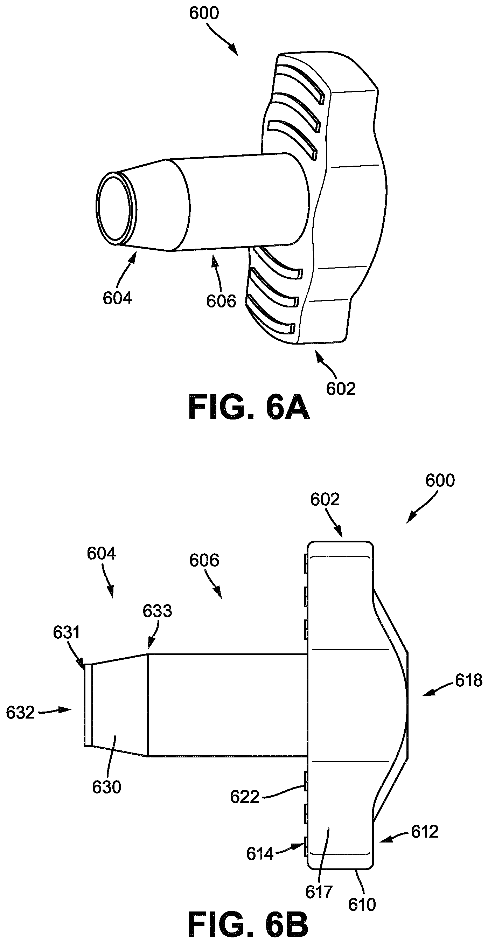

FIGS. 6A-6E illustrate another example of a transfer sleeve 600 in accordance with an embodiment hereof. One skilled in the art will realize that FIGS. 6A-6E illustrate one example of a transfer sleeve and that existing components illustrated in FIGS. 6A-6E may be removed and/or additional components may be added to the transfer sleeve 600.

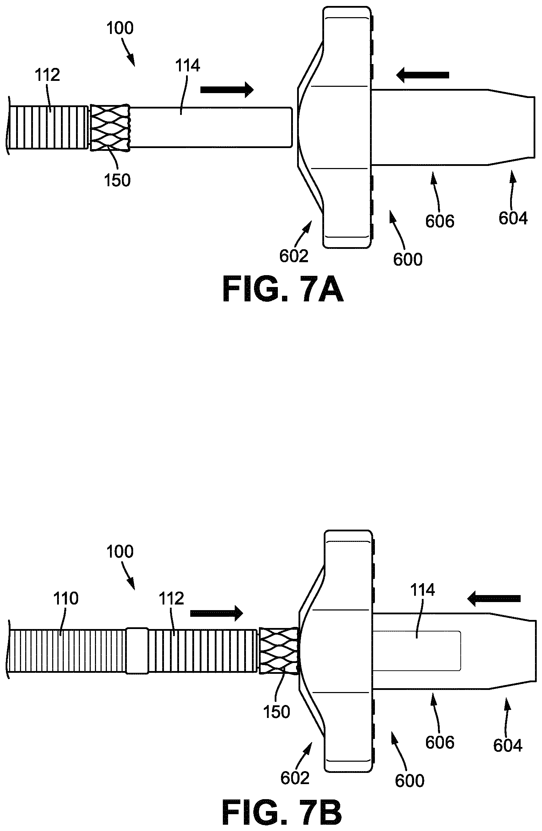

As illustrated in FIGS. 6A and 6B, which are perspective and side views of the transfer sleeve 600, respectively, the transfer sleeve 600 includes a proximal portion 602, a distal portion 604, and a body portion 606 coupled between the proximal portion 602 and the distal portion 604. In embodiments, the transfer sleeve 600, *e.g.,* the proximal portion 602, the distal portion 604, and the body portion 606 can be constructed as a single integrated structure or piece. For example, the transfer sleeve 600, *e.g.,* the proximal portion 602, the distal portion 604, and the body portion 606 can be constructed using manufacturing processes, such as injection molding, to form the single integrated structure or piece. The transfer sleeve 600 is configured to be positioned over a distal portion of a delivery device, *e.g.,* the distal portion 104 of the delivery system 100. For example, the transfer sleeve 600 can be installed over the implantable medical device 150 that is installed on the delivery system 100, as described in further detail below with reference FIGS. 4 and 7A-7H. When installed, the proximal portion 602 is positioned to be on the proximal side of the implantable medical device 150, and the distal portion 604 is positioned on the distal side of the implantable medical device 150 such that the implantable medical device 150 is located within the body portion 606. The proximal portion 602 seals to a first portion of the delivery system 100, *e.g.,* the inner shaft 110, and the distal portion 604 seals to a second portion of the delivery system 100, *e.g.,* the capsule 114, thereby providing a sterile environment for the implantable medical device 150.

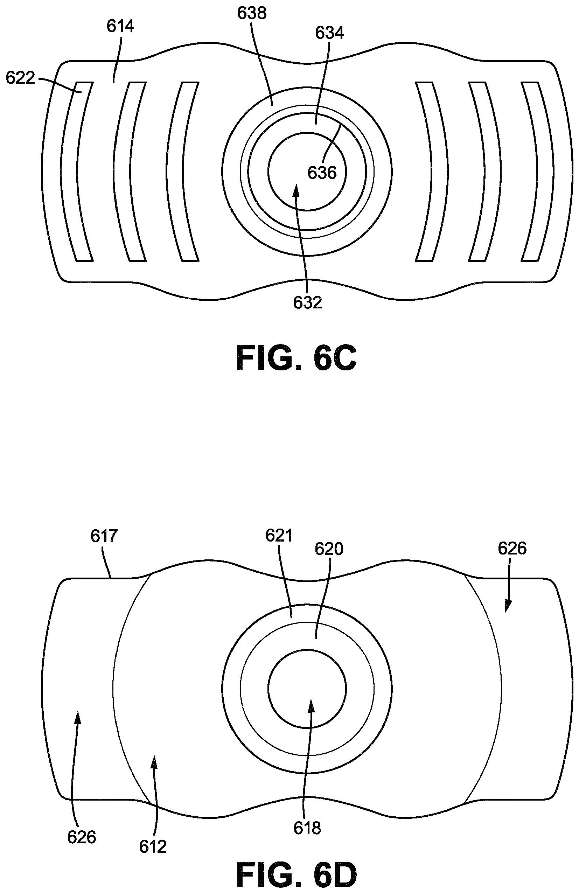

As illustrated in FIG. 6B, the proximal portion 602 may include a hub 610 that includes a proximal side 612, a distal side 614, and a body 617 extending from the proximal side 612 to the distal side 614. The distal portion 604 may include a tapered seal 630. As illustrated in FIG. 6C, which is a view from the distal end of the transfer sleeve 600, the tapered seal 630 may be formed as a tubular structure that defines a channel 632 that extends along the central longitudinal axis of the tapered seal 630 from a distal end 631 to a connection 633 with the body portion 606. The tapered seal 630 can be formed having a tapered outer surface 638 that decreases in outer diameter from the connection 633 with the body portion 604 to the distal end 631 of the tapered seal 630. In embodiments, the seal clamp 612, the tapered seal 630, and the body portion 106 can be formed of a rigid or semi-rigid material, *e.g.,* silicon, rubber, latex, polymeric material, *etc.*

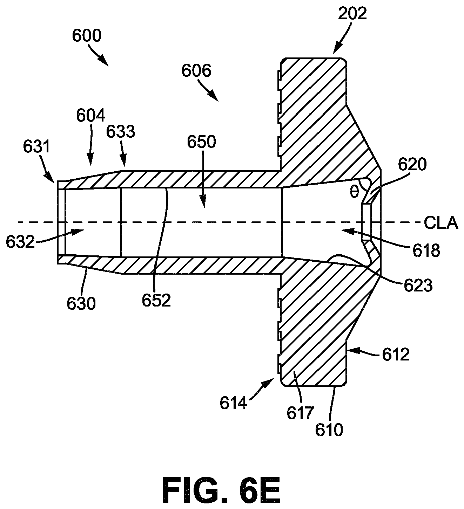

In embodiments, the channel 632 can be formed having a circular cross-section that decreases in diameter, thereby forming a channel from the distal end 631 to the connection 633 having a frustoconical shape. As illustrated in FIG. 6E, which is a cross-section view, an inner diameter of the channel 632 can decrease from the connection 633 to the distal end 631, thereby forming a radial seal 634. That is, the sidewall 636 decreases in diameter inward towards a central longitudinal axis CLA thereby forming the frustoconical shape of the tapered seal 630 that operates as the radial seal 634. The tapered seal 630 is configured to form a seal with components of a delivery system when the transfer sleeve 600 is installed on the delivery system.

As illustrated in FIG. 6B and FIG. 6C, the distal side 614 of the hub 610 may include a number of curved ridges 622 that extend from the distal side 614. The ridges 622 can operate to assist with gripping, handling, and maneuvering of the transfer sleeve 600. As illustrated in FIG. 6D, which is a view of the proximal side 612, the hub 610 may be formed having a solid body that includes a channel 618 that extends from the proximal side 612 to the distal side 614 of the hub 610. An outer surface 619 of the body 617 can be constructed to form two tabs 626 that extend from opposing sides of the hub 610. While FIG. 6D illustrates the hub 610 including the two tabs 626, in some embodiments, the outer surface 617 of the hub 610 can be formed having a circular cross-section thereby defining a cylindrical shape for the hub 610. The hub 610 can be formed of a rigid or semi-rigid material, *e.g.,* silicon, rubber, latex, polymeric material, *etc.* The hub 610 can also include a circular ridge 621 that extends from the proximal side 612, as further illustrated in FIG. 6E.

In embodiments, the channel 618 can be formed having a circular cross-section that decreases in diameter, thereby forming a channel from the proximal side 612 to the distal side 614 having a frustoconical shape. As illustrated in FIG. 6E, an inner diameter of the channel 618 can decrease from the connection proximal side 612 to the distal side 614, thereby creating a taper in the channel 618. A radial seal 620 may be positioned inside the channel 618. The radial seal 620 may be coupled to a sidewall 623 defining the channel 618 formed within the hub 610. In some embodiments, the radial seal 620 can be constructed as an integrated component of the hub 610. The radial seal 620 extends inward from the sidewall 623 towards a central axis of the hub 610. In some embodiments, as illustrated in FIG. 6E, radial seal 620 can extend at an angle, *θ*, from the sidewall 623 towards a central longitudinal axis of the hub 610. The radial seal 620 is configured to form a seal with components of a delivery system when the transfer sleeve 600 is installed on the delivery system. The radial seal 620 can be formed of a flexible material, *e.g*., silicon, rubber, latex, or other polymeric flexible material, that expands, contracts, and moves when components of a delivery system are inserted through the channel 618.

In any embodiment, the tabs 626 may extend from opposing sides of the hub 610 to a length that enables the hub 610 to engage with components of an introducer sheath, e.g., the handle portion 162 of the introducer sheath 160. As such, as a catheter portion 102 is inserted through the introducer sheath 160, the handle portion 162 applies a force on the transfer sleeve 300 thereby preventing the transfer sleeve 300 from moving as the catheter portion 102 is advanced distally, thereby causing the transfer sleeve 300 to slide along the catheter portion 102 to a more proximal position on the catheter portion 102, as described below in further detail. For example, each of the tabs 626 can extend approximately 50.0 mm from a central axis of the transfer sleeve 600.

In any embodiment, the channel 618 and the channel 632 can be constructed having diameters that allow components of a delivery system, *e.g*., the outer shaft 108, the inner shaft 110, and the capsule 114 of the delivery system 100, to pass through the channel 618 and the channel 632, while still engaging with the radial seal 620 and the radial seal 634 formed by the tapered seal 630. For example, the diameter of the channel 632 at the connection 633 can be approximately 10.40 mm, and the diameter of the distal end 631 can be approximately 8.95 mm. Additionally, for example, the diameter of the channel 618 at the proximal surface 612 can be approximately 13.8 mm, and the diameter of the channel 618 at the distal surface can be approximately 10.40 mm.

As illustrated in FIG. 6E, the body portion 606 can be constructed as a cylindrical tube having an inner sidewall 652 that forms a channel 650. In any embodiment, the channel 650 can be constructed having a diameter that allows components of a delivery system, e.g., the outer shaft 108, the inner shaft 110, and the capsule 114 of the delivery system 100, to pass through the channel 650. For example, the diameter of the channel 650 can be approximately 10.40 mm. Moreover, in embodiments, the channel 650 can be constructed to a length that allow the hub 610 and the tapered seal 630 to engage with components of the delivery system 100 on either side of an exposed portion 152 of the implantable medical device thereby containing the exposed portion 152 with in the channel 650. For example, a length of the cavity 252 can be approximately 24.5 mm. Additionally, for example, a length of the transfer sleeve from the proximal end to the distal end can be approximately 50 mm. While several examples of dimension as described above, the dimensions of the transfer sleeve 600 can be formed to larger and/or smaller dimension based on dimensions of the delivery system 100, the introducer sheath, 160, and/or the implantable medical device 100.

In embodiments, the channel 618 of the hub 610 and the channel 632 of the tapered seal 630 are in fluid communication with the channel 650. When the radial seal 620 of the hub 610 and the radial seal 632 formed by the tapered seal 630 engage with components of the delivery system 100, the cavity channel 650 forms a fluid tight chamber in which exposed portions of the implantable medical device 150 can be contained. The body portion 606 can be constructed of any type of rigid or semi-rigid material, *e.g*., silicon, rubber, latex, polymeric material, *etc.* In some embodiments, any one of the hub 610, the tapered seal 630, and the body portion 606 can be transparent or semi-transparent thereby providing a view or partial view of the channel 618, channel 632, and channel 650, respectively. In some embodiments, the hub 610, the tapered seal 630, and the body portion 606 can be constructed as a single integrated piece. For example, the 610, the body portion 606, and the tapered seal 630 (and the components of each) can be formed as a single structure by a process such as injection molding.

In embodiments, the method 400 described above can be performed using the transfer sleeve 600 to provide a sterile environment for an implantable medical device. The operation of the method 400 with the transfer sleeve 600 will be described with reference to FIG. 4 and FIGS. 7A-7I. One skilled in the art will realize that FIGS. 7A-7I illustrate one example of a method using a transfer sleeve and that operations illustrated in FIG. 4 may be removed and/or additional operations may be added to the method 400. One skilled in the art will also realize that the installation of the transfer sleeve 600 onto the delivery system can be performed while portions of the delivery system containing the implantable medical device are submerged in a sterile bath.

In step 402, a transfer sleeve can be inserted into over a distal end of a delivery system. For example, as illustrated in FIG. 7A, which illustrates the method 400 being performed using the transfer sleeve 600 and the delivery system 100, the transfer sleeve 600 can be positioned so that the proximal portion 602 is facing the distal end (the capsule 114) of the catheter portion 102 (the distal portion 104). Once positioned, the transfer sleeve 600 and the distal portion 104 can be advanced towards one another, e.g., the transfer sleeve 600 is advanced in a proximal direction towards the capsule 114 and/or the capsule 114 is advanced towards the proximal portion 602. The capsule 114 of the distal portion 104 can be inserted into the proximal portion 602 of the transfer sleeve 600. That is, the capsule 114 of the catheter portion 102 can be inserted into the channel 618 of the hub 610. As the capsule 114 enters channel 618, the radial seal 620 engages with the outer surfaces of the capsule 114 creating a fluid tight seal between the capsule 114 and the hub 610.

In step 404, the transfer sleeve can be advanced over a capsule of the delivery system. For example, as illustrated in FIG. 7B, the transfer sleeve 600 and the catheter portion 102 of the delivery system 100 continue to be advanced towards one another until the capsule 114 passes into the body portion 606 of the transfer sleeve 600. Then, the transfer sleeve 600 and the catheter portion 102 of the delivery system 100 continue to be advanced towards one another until the capsule 114 enters the channel 632 of the tapered seal 630.

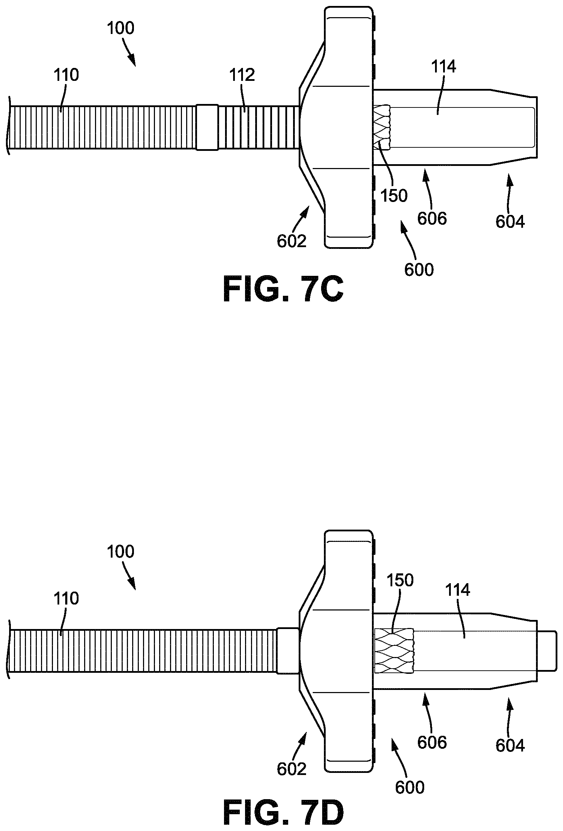

In step 406, the transfer sleeve can be positioned over the implantable medical device. For example, as illustrated in FIG. 7C, the advancement of the transfer sleeve 600 is continued until the hub 610 is positioned on the inner shaft 110 and the capsule 114 extends from the tapered seal 630. The transfer sleeve 600 is positioned so that any exposed portion of the implantable medical device 150 is within the channel 650 of the body portion 606, as illustrated in FIG. 7D. Because the channel 650 is fluid tight, sterile fluid utilized during the installation of the transfer sleeve 600, e.g., sterile fluid bath, is contained within the channel 650 thereby providing a sterile environment for any exposed portion of the implantable medical device 150.

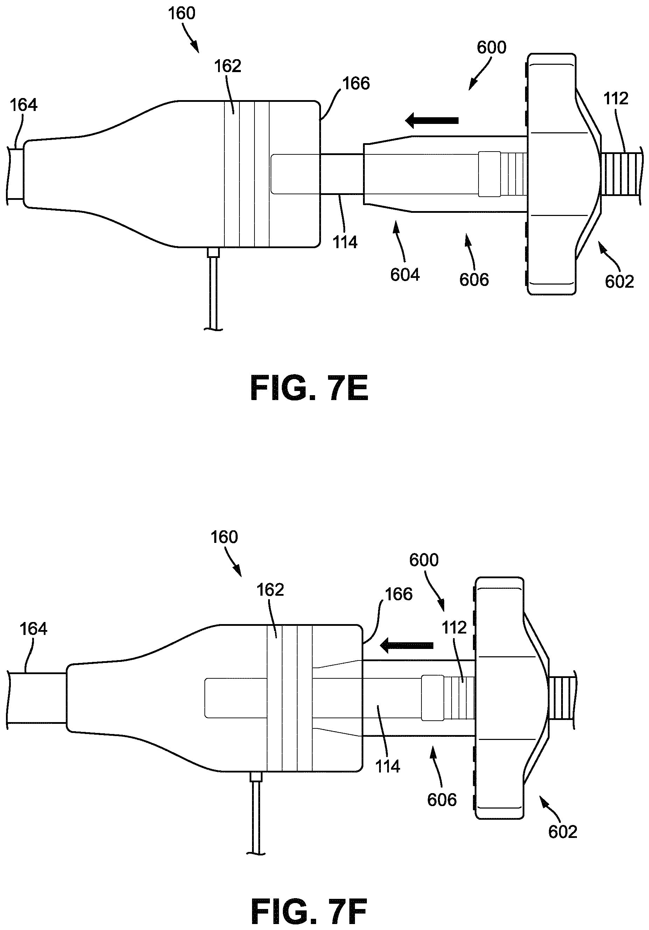

In step 408, the distal end of the delivery system can be inserted into an introducer sheath. For example, once the implantable medical device 150 is ready for delivery to an implant site, the distal end 104 of the catheter portion 102 can be inserted into the introducer sheath 160 to introduce the catheter portion 102 to a patient's vasculature. While not shown, prior to insertion of the catheter portion 102, the introducer sheath 160 can be installed in a vessel of a patient using known techniques, *e.g*., puncture needle and dilator. As illustrated in FIG. 7E, the distal end 104 of delivery system 100 (including the transfer sleeve 600) is advanced towards the handle portion 162 of the introducer sheath 160. The distal end 104 of the delivery system 100 is advanced until the capsule 114 enters the central lumen of the handle portion 162.

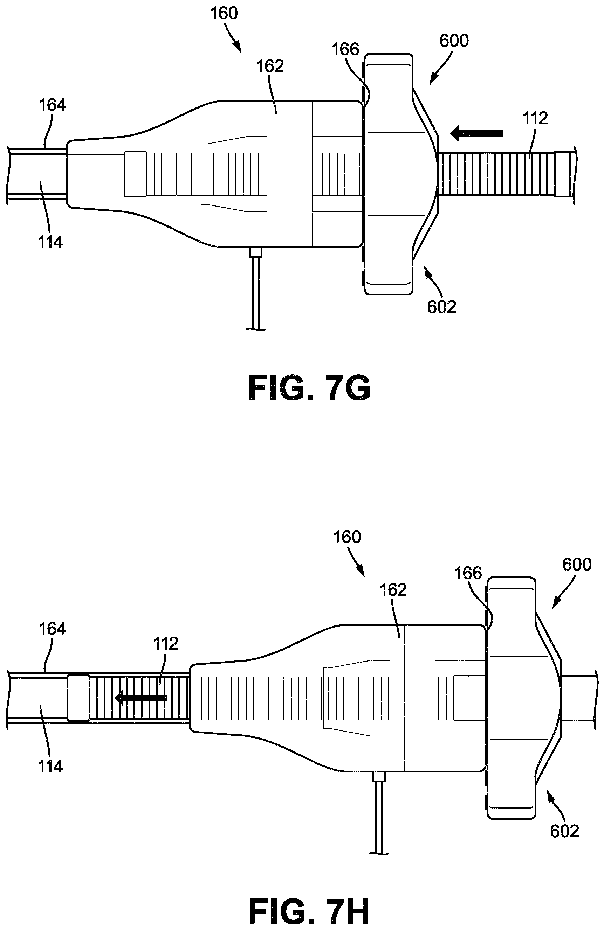

In step 410, the delivery system can be advanced through the introducer sheath. The distal end 104 of the delivery system 100 is advanced so that the capsule 114 enters the central lumen of the sheath portion 164 of the introducer sheath 160. As illustrated in FIG. 7F, the distal end 104 of delivery system 100 (including the transfer sleeve 600) is further advanced so that the distal end 604 (the tapered seal 630) and the body portion 606 enter the central lumen of the handle portion 162. As illustrated in Fig. 7G, the distal end 104 of delivery system 100 (including the transfer sleeve 600) is advanced until the proximal portion 602 (the tabs 626 of the hub 610) abuts the handle portion 162 of the introducer sheath 160.

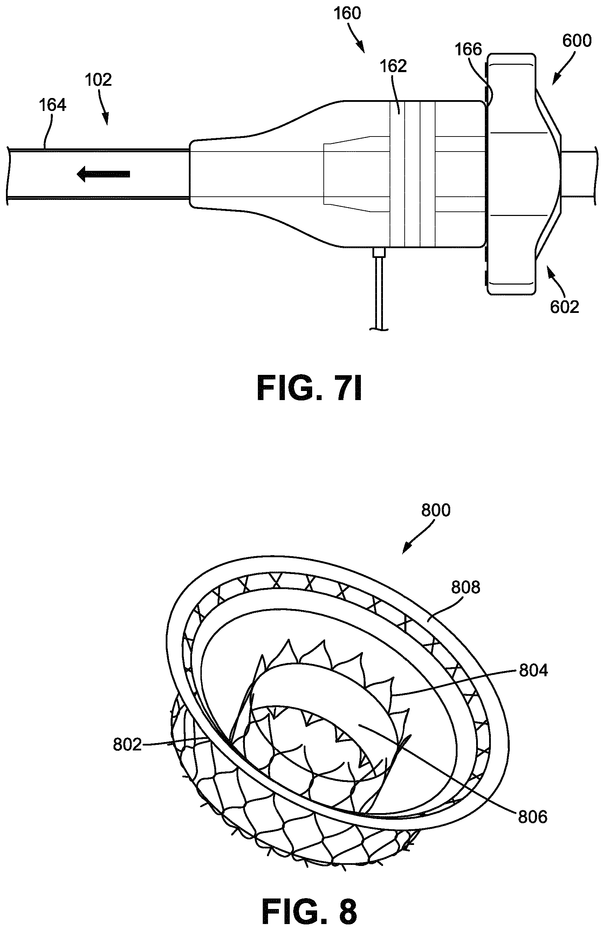

As the catheter portion 102 is advanced through the introducer sheath 160, the handle portion 162 applies a force on the proximal portion 602 (the tabs 626 of the hub 610) thereby preventing the transfer sleeve 600 from moving distally with the catheter portion 102 of the delivery system. Therefore, the transfer sleeve 600 moves in a proximal direction relative to the catheter portion 102 of the delivery system 100, as illustrated in FIG. 7H and 7I. As the inner shaft 110 moves distally through the transfer sleeve 600, the implantable medical device 150 passes out of the transfer sleeve 600 into the introducer sheath 160. Because the transfer sleeve 600 (the tabs 626) abuts the handle portion 162, the implantable medical device 150 is only exposed to the central lumens of the introducer shaft 160 thereby maintaining a sterile environment for the implantable medical device 150. In some embodiments, the transfer sleeve 600 can remain on the inner shaft 110 of the catheter portion 102 as the inner shaft 110 is advanced through the introducer sheath 160. In some embodiments, the catheter portion 102 can advanced distally until the transfer sleeve 600 is positioned on the outer shaft 108 of the catheter portion 102.

In embodiments, the implantable medical devices useful with the present disclosure can be a prosthetic valve such as the prosthetic valve sold under the trade name Intrepid^{™} available from Medtronic, Inc., and the like. A non-limiting example of an implantable medical device useful with systems, devices and methods of the present disclosure is illustrated in FIG. 8. In particular, FIG. 8 illustrates a side view of a prosthetic heart valve 800 in a normal or expanded (uncompressed) arrangement. The prosthetic heart valve 800 includes an outer stent or frame 802 and an inner stent or frame 804. The outer stent 802 is configured to engage an annulus and leaflets on a native anatomy, *e.g*., a heart valve. The outer stent 802 is configured to provide fixation to the native heart valve while isolating the inner stent 804 from the native anatomy. A flexible brim 808 is coupled to the outer stent 802. The flexible brim 808 is configured to seat against the atrial side of a native mitral valve annulus.

The inner stent 804 is configured to house a valve structure 806. The inner stent 804 and the outer stent 802 are generally constructed so as to be expandable from the compressed arrangement to the uncompressed arrangement. In some embodiments, the inner stent 804 and the outer stent 802 are self-expanding. In other embodiments, the inner stent 804 and the outer stent 802 can be designed to the expanded arrangement by a separate device (*e.g.,* a balloon). The valve structure 806 is attached to the inner stent 804 and provides two or more (typically three) leaflets. The valve structure 806 can be attached to the inner stent 804 in various manners, such as by sewing the valve structure 806 to one or more of the wire segments or commissure posts defined by the inner stent 804.

The prosthetic heart valve 800 can be configured to replace or repair a mitral valve. Alternatively, other shapes are also envisioned, adapted to the specific anatomy of the valve to be repaired (e.g., stented prosthetic heart valves in accordance with the present disclosure can be shaped and/or sized for replacing a native aortic, pulmonic, or tricuspid valve). A wide variety of other constructions are also acceptable and within the scope of the present disclosure.

In embodiments, the inner stent 804 and the outer stent 802 can be formed from a shape memory material such as a nickel titanium alloy (e.g., Nitinol). With this material, the support structure is self-expandable from the compressed arrangement to the normal, expanded arrangement, such as by the application of heat, energy, and the like, or by the removal of external forces (*e.g*., compressive forces). Thus, the inner stent 804 and the outer stent 802 can also be compressed and re-expanded multiple times without significantly damaging the structure of the stent frame. In addition, the inner stent 804 and the outer stent 802 may be laser-cut from a single piece of material or may be assembled from a number of different components or manufactured from a various other methods known in the art.

In embodiments, the inner stent 804 can generally a tubular support structure having an internal area in which the leaflets of the valve structure 806 can be secured. The leaflets of the valve structure 806 can be formed from a variety of materials, such as autologous tissue, xenograph material, or synthetics as are known in the art. In some embodiments, the leaflets of the valve structure 806 may be provided as a homogenous, biological valve structure, such as porcine, bovine, or equine valves. In some embodiments, the leaflets of the valve structure 806 can be provided independent of one another and subsequently assembled to the support structure of the inner stent 804.

It should be understood that various embodiments disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). Each embodiment and each aspect so defined may be combined with any other embodiment or with any other aspect unless clearly indicated to the contrary. In addition, while certain aspects of this disclosure are described as being performed by a single device or component for purposes of clarity, it should be understood that the techniques of this disclosure may be performed by a combination of devices or components associated with, for example, a medical device.

## Claims

1. A transfer sleeve (200, 300) for use with a delivery system (100) for an implantable medical device, the transfer sleeve comprising:
a proximal portion (202, 302) defining a proximal channel, wherein the proximal channel has a cross-sectional area that allows a shaft (108, 110) of the delivery system to fit within the proximal channel of the proximal portion;
a distal portion (204, 304) defining a distal channel, wherein the distal channel has a cross-section area that allows the shaft of the delivery system to fit within the distal channel of the distal portion; and
a body portion (206, 306) coupled between the proximal portion and the distal portion and defining a body cavity, wherein:
the body portion is formed of a compressible material that allows the proximal portion to move axially relative to the distal portion, and
the body cavity, proximal channel, and distal channel form a fluid tight cavity when the proximal portion is engaged with the shaft of the delivery system and the distal portion is engaged with a capsule (114).

2. The transfer sleeve of claim 1, wherein the proximal portion further comprises:
a first seal clamp (212, 310); and
a first radial seal (220, 320) coupled to an interior surface of the first seal clamp; wherein the first radial seal forms a fluid seal with the shaft when the proximal portion is engaged with the shaft.

3. The transfer sleeve of claim 1 or 2, wherein the distal portion further comprises:
a second seal clamp (230, 312); and
a second radial seal (240, 320) coupled to an interior surface of the second seal clamp; wherein the second radial seal forms a fluid seal with the capsule when the proximal portion is engaged with the capsule.

4. The transfer sleeve of claim 3, wherein the first seal clamp and the second seal clamp are tubular shaped and wherein the second seal clamp has an outer diameter formed to a size to engage with an edge of an introducer sheath (160) when the delivery system is inserted into the introducer sheath thereby causing the second seal clamp to move towards a proximal end of the delivery system.

5. The transfer sleeve of any one of claims 1 to 4, wherein the body portion is formed of an elastic material that compresses when the second seal clamp moves towards the proximal end of the delivery system; and optionally wherein the elastic material is latex.

6. The transfer sleeve of any one of claims 1 to 5, wherein the body portion further comprises:
a plurality of circular spines (352) positioned axially between the proximal portion and the distal portion; and
a plurality of compressible membranes (354), one of the plurality of compressible membranes coupled between adjacent circular spines from the plurality of circular spines, wherein the plurality of compressible membranes compresses when the second seal clamp moves towards the proximal end of the delivery system.

7. The transfer sleeve of any one of claims 1 to 6, wherein at least one of the body portion, proximal portion, or distal portion is constructed of a transparent material or semi-transparent material.

## Patentansprüche

1. Übertragungshülse (200, 300) zur Verwendung mit einem Abgabesystem (100) für eine implantierbare medizinische Vorrichtung, die Übertragungshülse umfassend:
einen proximalen Abschnitt (202, 302), der einen proximalen Kanal definiert, wobei der proximale Kanal einen Querschnittsbereich aufweist, der es ermöglicht, dass ein Schaft (108, 110) des Abgabesystems innerhalb des proximalen Kanals des proximalen Abschnitts passt;
einen distalen Abschnitt (204, 304), der einen distalen Kanal definiert, wobei der distale Kanal einen Querschnittsbereich aufweist, der es ermöglicht, dass der Schaft des Abgabesystems innerhalb des distalen Kanals des distalen Abschnitts passt; und
einen Körperabschnitt (206, 306), der zwischen dem proximalen Abschnitt und dem distalen Abschnitt gekoppelt ist und einen Körperhohlraum definiert, wobei:
der Körperabschnitt aus einem komprimierbaren Material, das es ermöglicht, dass sich der proximale Abschnitt relativ zu dem distalen Abschnitt axial bewegt, ausgebildet ist, und
der Körperhohlraum, der proximale Kanal und der distale Kanal einen fluiddichten Hohlraum ausbilden, wenn der proximale Abschnitt den Schaft des Abgabesystems in Eingriff nimmt und der distale Abschnitt eine Kapsel (114) in Eingriff nimmt.

2. Übertragungshülse nach Anspruch 1, wobei der proximale Abschnitt ferner umfasst:
eine erste Dichtungsklemme (212, 310); und
eine erste Radialdichtung (220, 320), die mit einer Innenoberfläche der ersten Dichtungsklemme gekoppelt ist; wobei die erste Radialdichtung eine Fluiddichtung mit dem Schaft ausbildet, wenn der proximale Abschnitt den Schaft in Eingriff nimmt.

3. Übertragungshülse nach Anspruch 1 oder 2, wobei der distale Abschnitt ferner umfasst:
eine zweite Dichtungsklemme (230, 312); und
eine zweite Radialdichtung (240, 320), die mit einer Innenoberfläche der zweiten Dichtungsklemme gekoppelt ist; wobei die zweite Radialdichtung eine Fluiddichtung mit der Kapsel ausbildet, wenn der proximale Abschnitt die Kapsel in Eingriff nimmt.

4. Übertragungshülse nach Anspruch 3, wobei die erste Dichtungsklemme und die zweite Dichtungsklemme röhrchenförmig geformt sind, und wobei die zweite Dichtungsklemme einen Außendurchmesser aufweist, der zu einer Größe ausgebildet ist, um einen Rand einer Einführhülse (160) in Eingriff zu nehmen, wenn das Abgabesystem in die Einführhülse eingeführt wird, wobei dadurch die zweite Dichtungsklemme veranlasst wird, sich zu einem proximalen Ende des Abgabesystems zu bewegen.

5. Übertragungshülse nach einem der Ansprüche 1 bis 4, wobei der Körperabschnitt aus einem elastischen Material, das sich komprimiert, wenn sich die zweite Dichtungsklemme zu dem proximalen Ende des Abgabesystems bewegt, ausgebildet ist; und optional wobei das elastische Material Latex ist.

6. Übertragungshülse nach einem der Ansprüche 1 bis 5, wobei der Körperabschnitt ferner umfasst:
eine Vielzahl von kreisförmigen Stacheln (352), die zwischen dem proximalen Abschnitt und dem distalen Abschnitt axial positioniert sind; und
eine Vielzahl von komprimierbaren Membranen (354), wobei eine der Vielzahl von komprimierbaren Membranen zwischen angrenzenden kreisförmigen Stacheln aus der Vielzahl von kreisförmigen Stacheln gekoppelt ist, wobei sich die Vielzahl von komprimierbaren Membranen komprimiert, wenn sich die zweite Dichtungsklemme zu dem proximalen Ende des Abgabesystems bewegt.

7. Übertragungshülse nach einem der Ansprüche 1 bis 6, wobei mindestens einer von dem Körperabschnitt, dem proximalen Abschnitt oder dem distalen Abschnitt aus einem transparenten Material oder einem halbtransparenten Material konstruiert ist.

## Revendications

1. Manchon de transfert (200, 300) pour une utilisation avec un système d'administration (100) pour un dispositif médical implantable, le manchon de transfert comprenant :
une partie distale (202, 302) définissant un canal proximal, dans lequel le canal proximal présente une zone de section transversale qui permet à une tige (108, 110) du système d'administration de s'ajuster dans le canal proximal de la partie proximale ;
une partie distale (204, 304) définissant un canal distal, dans lequel le canal distal présente une zone de section transversale qui permet à la tige du système d'administration de s'ajuster dans le canal distal de la partie distale ; et
une partie de corps (206, 306) accouplée entre la partie proximale et la partie distale et définissant une cavité de corps, dans lequel :
la partie de corps est formée d'un matériau compressible qui permet à la partie proximale de se déplacer axialement par rapport à la partie distale, et
la cavité de corps, le canal proximal et le canal distal forment une cavité imperméable aux fluides lorsque la partie proximale vient en prise avec la tige du système d'administration et que la partie distale vient en prise avec une capsule (114).

2. Manchon de transfert selon la revendication 1, dans lequel la partie proximale comprend en outre :
un premier collier de serrage (212, 310) ; et
un premier joint radial (220, 320) accouplé à une surface intérieure du premier collier de serrage ; dans lequel le premier joint radial forme un joint d'étanchéité avec la tige lorsque la partie proximale vient en prise avec la tige.

3. Manchon de transfert selon la revendication 1 ou 2, dans lequel la partie distale comprend en outre :
un second collier de serrage (230, 312) ; et
un second joint radial (240, 320) accouplé à une surface intérieure du second collier de serrage ; dans lequel le second joint radial forme un joint d'étanchéité avec la capsule lorsque la partie proximale vient en prise avec la capsule.

4. Manchon de transfert selon la revendication 3, dans lequel le premier collier de serrage et le second collier de serrage sont de forme tubulaire et dans lequel le second collier de serrage a un diamètre extérieur formé à une taille suffisante pour venir en prise avec un bord d'une gaine d'introducteur (160) lorsque le système d'administration est inséré dans la gaine d'introducteur, amenant ainsi le second collier de serrage à se déplacer vers une extrémité proximale du système d'administration.

5. Manchon de transfert selon l'une quelconque des revendications 1 à 4, dans lequel la partie de corps est formée d'un matériau élastique qui se comprime lorsque le second collier de serrage se déplace vers l'extrémité proximale du système d'administration ; et éventuellement dans lequel le matériau élastique est du latex.

6. Manchon de transfert selon l'une quelconque des revendications 1 à 5, dans lequel la partie de corps comprend en outre :
une pluralité de supports circulaires (352) positionnés axialement entre la partie proximale et la partie distale ; et
une pluralité de membranes compressibles (354), l'une de la pluralité de membranes compressibles étant accouplée entre des supports circulaires adjacents à partir de la pluralité de supports circulaires, dans lequel la pluralité de membranes compressibles se comprime lorsque le second collier de serrage se déplace vers l'extrémité proximale du système d'administration.

7. Manchon de transfert selon l'une quelconque des revendications 1 à 6, dans lequel au moins l'une parmi la partie de corps, la partie proximale ou la partie distale est constituée d'un matériau transparent ou d'un matériau semi-transparent.
